(19) Europäisches Patentamt
European Patent Office
Office européen des brevets

(11) Veröffentlichungsnummer: **0 090 269**
**A1**

# (12) EUROPÄISCHE PATENTANMELDUNG

(21) Anmeldenummer: **83102618.2**

(22) Anmeldetag: **17.03.83**

(51) Int. Cl.³: **C 07 D 249/08**, C 07 D 233/56, C 07 D 231/12, A 01 N 43/50, A 01 N 43/56, A 01 N 43/64

(30) Priorität: **23.03.82 DE 3210570**

(43) Veröffentlichungstag der Anmeldung: **05.10.83 Patentblatt 83/40**

(84) Benannte Vertragsstaaten: **AT BE CH DE FR GB IT LI NL SE**

(71) Anmelder: **HOECHST AKTIENGESELLSCHAFT, Postfach 80 03 20, D-6230 Frankfurt am Main 80 (DE)**

(72) Erfinder: **Ehrhardt, Heinz, Dr., Bergstrasse 21, D-8901 Rehling (DE)**
Erfinder: **Mildenberger, Hilmar, Dr., Fasanenstrasse 24, D-6233 Kelkheim (Taunus) (DE)**
Erfinder: **Sachse, Burkhard, Dr., An der Ziegelei 30, D-6233 Kelkheim (Taunus) (DE)**
Erfinder: **Hartz, Peter, Dr., An der Ziegelei 28, D-6233 Kelkheim (Taunus) (DE)**

(54) **3-Azolyl-1,2-diaryl-1-halogen-1-propene, ihre Herstellung, ihre Verwendung als Pflanzenschutzmittel und diese Verbindungen enthaltende Präparate.**

(57) Die Erfindung betrifft 3-Azolyl-1,2-diaryl-1-halogen-1-propene der Formel

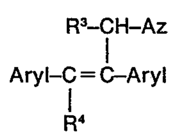

in der $R^4$ Chlor oder Brom und Az ein 1,2,4-Triazol-1-yl-, ein Imidazol-1-yl oder ein Pyrazol-1-ylrest ist, sowie deren Herstellung.

Die Verbindungen finden Verwendung als Biozide, insbesondere Fungizide, z.B. für die Bekämpfung von Echtem Mehltau.

EP 0 090 269 A1

HOECHST AKTIENGESELLSCHAFT HOE 82/F 800

0090269

Dr.Mb/Lr

# 3-Azolyl-1,2-diaryl-1-halogen-1-propene, ihre Herstellung, ihre Verwendung als Pflanzenschutzmittel und diese Verbindungen enthaltende Präparate

Es ist bekannt, daß 1,2-Diaryl-3-triazolyl-1-propene fungizide Eigenschaften besitzen ( DE-OS 26 52 313). Für ihren Einsatz in der Praxis hat sich allerdings als nachteilig erwiesen, daß bei niederen Aufwandmengen ihre Aktivität gegen Rost- und Mehltaupilze unzureichend und ihre Wirkungsbreite zu gering ist.

Es wurde gefunden, daß 1,2-Diaryl-3-azolylpropene, bei denen das C-Atom 1 der Propenkette ein Halogenatom trägt, und die somit als 3-Azolyl-1,2-diaryl-1-halogen-1-propene bezeichnet werden, überraschenderweise eine erheblich höhere fungizide und bakterizide Potenz gegen Rost- und Mehltaupilze aufweisen als die obengenannten Verbindungen und gleichzeitig eine Reihe weiterer pilzlicher Erkrankungen in hervorragender Weise bekämpfen.

Die vorliegende Erfindung betrifft daher bisher nicht bekannte 3-Azolyl-1,2-diaryl-1-halogen-1-propene der Formel (I)

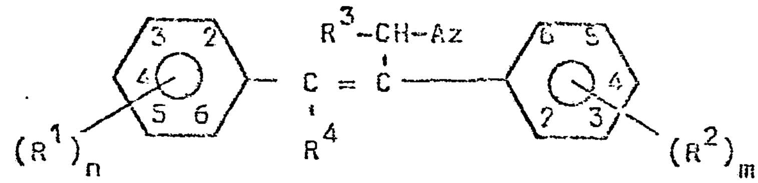

(I),

in welcher

$R^1$ und $R^2$ unabhängig voneinander für Wasserstoff, Halogen, $-CF_3$, $-OCF_2CF_2H$, $C_1$- bis $C_8$-, vorzugsweise $C_1$- und $C_2$-Alkyl, $C_5$- oder $C_6$-Cycloalkyl, $C_1$- bis $C_6$-,

BAD ORIGINAL

vorzugsweise $C_1$- und $C_2$-Alkoxi, $C_2$- bis $C_6$-, vorzugsweise $C_3$-Alkenoxi, oder auch für Phenoxi oder Phenyl und die Halogensubstitutionsprodukte der beiden letztgenannten Reste, vorzugsweise die mit 1 oder 2 Br- oder Cl-Atomen, stehen oder Reste der Formeln $-CH_2CH_2CH_2-$, $-CH_2CH_2CH_2CH_2-$, $-CH{=}CH{-}CH{=}CH-$, die die C-Atome der Ringpositionen 2 und 3 oder 3 und 4 miteinander verbinden, bedeuten.

Bevorzugt ist für $R^1$ Wasserstoff und Halogen und für $R^2$ Halogen und/oder Trifluormethyl.

$R^3$ ist Wasserstoff oder ein $C_1$- bis $C_5$-Alkylrest, bevorzugt Wasserstoff, und

$R^4$ steht für Chlor oder Brom, vorzugsweise Chlor.

n und m stehen für 1, 2 oder 3, vorzugsweise für 1 oder 2, wobei bei Mehrfachsubstitution die Substituenten auch unterschiedlich sein können.

Az hat die Bedeutung von 1,2,4-Triazol-1-yl, von Imidazol-1-yl oder von Pyrazol-1-yl.

Eingeschlossen sollen auch die Salze, Komplexsalze sowie Quaternisierungsprodukte der neuen Verbindungen sein.

Die Erfindung betrifft des weiteren ein Verfahren zur Herstellung der 3-Azolyl-1,2-diaryl-1-halogen-1-propene und ihre Verwendung als Biozide.

Man erhält die neuen Verbindungen, indem man 1 Mol eines 1,2-Diaryl-1,3-dihalogen-1-propens der allgemeinen Formel (II), in der Hal = Chlor oder Brom ist,

...

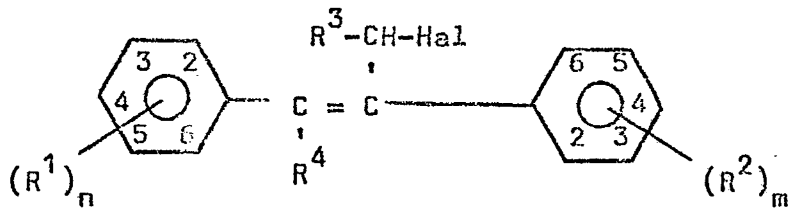

(II)

mit 1 bis 1,2 Mol 1,2,4-Triazol, Imidazol oder Pyrazol in Gegenwart eines Lösungsmittels und einer Base als Halogenwasserstoffacceptor bei Temperaturen zwischen 0 und 120, vorzugsweise 25 und 80 °C, umsetzt.

Geeignete Lösungsmittel sind bevorzugt aprotisch-dipolare, wie z. B. Acetonitril, Dimethylformamid, Dimethylsulfoxid und Tetramethylensulfon; besonders zu empfehlen sind Acetonitril und Dimethylformamid.

Als Base kann überschüssiges Azol, ein Alkali- oder Erdalkalihydroxid oder -carbonat oder ein tertiäres Amin verwendet werden. Bevorzugt ist Kaliumcarbonat. Die Base muß in mindestens molarer Menge, bezogen auf die Verbindung (II), eingesetzt werden.

Von den Ausgangsverbindungen der Formel (II) sind einige, und zwar solche, in denen Hal Brom ist, aus der DE-OS 21 03 119 bekannt. Diese und auch die noch nicht beschriebenen werden nach dem in jener OS angegebenen Verfahren hergestellt. Ausgangsverbindungen der Formel (II), in denen Hal Chlor ist, erhält man durch Umsetzen der entsprechenden Alkohole mit der 1,0- bis 1,5fach molaren Menge Thionylchlorid bei 0 bis 80 °C, wobei man in Gegenwart von Verdünnungsmitteln wie z. B. Tetrachlorkohlenstoff und/oder bei Anwesenheit von Pyridin in der 1- bis 10fachen Gewichtsmenge, bezogen auf den Alkohol, arbeiten kann.

Beispiele für Ausgangsverbindungen der Formel (II) sind:

1,3-Dichlor-1,2-diphenyl-1-propen, 1-Brom-3-chlor-1,2-diphenyl-1-propen, 1-Chlor-3-brom-1,2-diphenyl-1-propen, 1,3-Dichlor-1,2-

...

diphenyl-1-buten, 1,3-Dichlor-1-(4-methylphenyl)-2-phenyl-1-propen, 1-(4-Chlorphenyl)-1,3-dichlor-2-phenyl-1-propen, 2-(2-Chlorphenyl)-1,3-dichlor-1-phenyl-1-propen, 2-(3-Chlorphenyl)-1,3-dichlor-1-phenyl-1-propen, 2-(4-Chlorphenyl)-1,3-dichlor-1-phenyl-1-propen, 2-(2-Brom-4-chlorphenyl)-1,3-dichlor-1-phenyl-1-propen, 2-(4-Brom-2-chlor-phenyl)-1,3-dichlor-1-phenyl-1-propen, 1,3-Dichlor-2-(2,4-dichlorphenyl)-1-phenyl-propen, 1,3-Dichlor-2-(2,4-dichlorphenyl)-1-(4-chlorphenyl)-1-propen, 1,3-Dichlor-2-(3,4-dichlorphenyl)-1-phenyl-1-propen.

Die Isolierung der neuen Verbindungen, die im allgemeinen in sehr guter Ausbeute mit hohem Reinheitsgrad anfallen, erfolgt nach üblichen Methoden, eine gegebenenfalls gewünschte weitere Reinigung durch Destillation, Kristallisation oder über Salze.

Der Reaktionsablauf, der überraschend ist und nicht vorhersehbar war, da damit gerechnet werden mußte, daß beide Halogenatome des als Ausgangsmaterial dienenden 1,2-Diaryl-1,3-dihalogen-1-propens der Formel (II) reagieren würden - insbesondere dann, wenn es Bromatome sind, oder $R^4$ Brom und Hal Chlor ist - sei an der Umsetzung des E-1,3-Dichlor-1,2-diphenyl-1-propens mit 1,2,4-Triazol aufgezeigt:

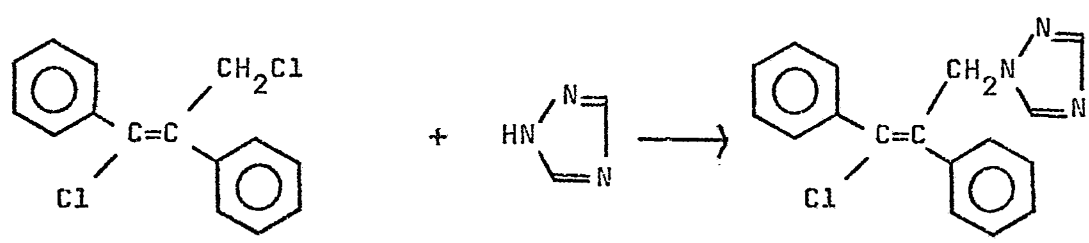

Die neuen 3-Azolyl-1,2-diaryl-1-halogen-1-propene, welche als E- und Z-Isomere -bevorzugt die erstgenannten - auftreten und synthetisiert werden, sind als basische Verbindungen zur Bildung von Salzen, Komplexsalzen und Quaternisierungsprodukten befähigt. Genannt seien Salze von organischen und anorganischen Säuren, wie Acetate, Fumarate, Oxalate, Benzoate usw., Nitrate

...

Bromide, Chloride, Sulfate, Salze der Naphthalinsulfonsäuren, Komplexe mit Metallen der Gruppen I b, II b, IV b oder VIII des Periodensystems, etwa Kupfer, Zink und Zinn und Quaternisierungsprodukte mit Alkyl- und Phenacylhalogeniden. Die Herstellung derartiger Derivate erfolgt nach den allgemein üblichen Methoden.

Die neuen Verbindungen sind, wie bereits erwähnt, als Biozide einsetzbar. Sie zeichnen sich insbesondere durch ihre hervorragende fungizide Wirksamkeit aus. Selbst bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der neuen Verbindungen erfaßt ferner eine Vielzahl verschiedener phytopathogener Pilze, wie z. B. Piricularia oryzae, Plasmopara viticola, verschiedene Rostarten, vor allem aber Venturia inaequalis, Cercospora beticola und Echte Mehltaupilze im Obst-, Gemüse-, Getreide- und Zierpflanzenbau.

Die Substanzen eignen sich ferner für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z.B.

...

polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Ihre Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitan-fettsäureester oder Polyoxethylen-sorbitester.

Stäubemittel werden durch Vermahlen des Wirkstoffes mit fein verteilten, festen Stoffen, z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

...

0090269

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 1 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z. B. Insektiziden, Akariziden, Herbiziden, Düngemitteln, Wachstumsregulatoren oder weiteren Fungiziden sind gegebenenfalls möglich, wobei u. U. auch synergistische Wirkungssteigerungen erzielt werden können.

Im folgenden seien einige Formulierungsbeispiele angeführt:

Ein Stäubemittel wird erhalten, indem man 10 Gewichtsteile Wirkstoff und 90 Gewichtsteile Talkum als Inertstoff mischt und in einer Schlagmühle zerkleinert.

...

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat stellt man her, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Ein emulgierbares Konzentrat läßt sich herstellen aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

Die folgenden Beispiele dienen der weiteren Erläuterung der Erfindung.

A. Herstellungsbeispiele

Beispiel 1

1-Chlor-1,2-diphenyl-3-(1,2,4-triazol-1-yl)-1-propen

Man gibt bei Raumtemperatur zu einer Mischung aus 7,59 g (0,11 Mol) 1,2,4-Triazol und 15,2 g (0,11 Mol) Kaliumcarbonat in 70 ml Dimethylformamid 26,3 g (0,1 Mol) 1,3-Dichlor-1,2-diphenyl-1-propen. Die Temperatur steigt bis 44 °C an, und es wird noch 3 Stunden bei 50 °C nachgerührt, worauf man auf Eis gießt und mit Dichlormethan ausschüttelt. Nach dem Ab-

BAD ORIGINAL

0090269

destillieren des Lösungsmittels verbleiben 27,2 g (92 %) einer farblosen Substanz vom Schmp. 123-4 °C.

| | | | | |
|---|---|---|---|---|
| $C_{17}H_{14}ClN_3$ (295,5) | ber.: | C 69,0 | H 4,7 | N 14,2 |
| | gef.: | C 68,3 | H 4,8 | N 14,1 |

IR (KBr): 3115, 3040 (CH), 1500, 1440, 1335, 1270, 1200, 1130, 1015, 890, 850, 780, 760, 745, 720, 680, 670, 650, 630 $cm^{-1}$

NMR ($CDCl_3$): $\delta$ = 4,95 (s, 2H, $CH_2$-), 7,00 - 7,60 (m, 12H, Ph + Triazol)

<u>Beispiele 2 bis 28</u>

Nach der in Beispiel 1 beschriebenen Methode wurden unter Einsatz nachfolgender Verbindungen weitere 3-Azolyl-1,2-diaryl-1-halogen-1-propene hergestellt.

Bei allen eingesetzten Verbindungen der Formel (II) war Hal = Chlor, ausgenommen bei der für Beispiel 11 verwendeten, wo Hal = Brom bedeutete.

...

| Beispiel Nr. | Ausgangsprodukte | | | | | Verfahrensprodukt |
|---|---|---|---|---|---|---|
| | 1,2-Diaryl-1,3-dihalogen-1-propen der Formel (II) | | | | Azol 1) | Schmp. bzw. Sdp. (°C) |
| | $(R^1)_n$ | $(R^2)_m$ | $R^3$ | $R^4$ | | |
| 2 | H | H | H | Cl | Im | 132-4 |
| 3 | H | H | H | Br | Tr | 98-105 |
| 4 | H | H | H | Br | Im | 195/0,03 |
| 5 | 4-$CH_3$ | H | H | Cl | Tr | 191-3/0,01 |
| 6 | 4-$CH_3$ | H | H | Cl | Im | 200-2/0,008 |
| 7 | 4-Cl | H | H | Cl | Tr | Oel |
| 8 | 4-Cl | H | H | Cl | Tr | 127-8 2) |
| 9 | 4-Cl | H | H | Cl | Im | Oel |
| 10 | H | 4-Cl | H | Cl | Tr | 198-200/0,03 |
| 11 | H | 4-Cl | H | Cl | Im | 212-4/0,03 |
| 12 | 4-Cl | 4-Cl | H | Cl | Tr | Oel |
| 13 | 4-Cl | 4-Cl | H | Cl | Im | Oel |
| 14 | 4-$CH_3$ | 4-Cl | H | Cl | Tr | 260 (Z) 3) |
| 15 | 4-$CH_3$ | 4-Cl | H | Cl | Im | 217 (Z) 3) |
| 16 | 4-$CH_3$ | 4-Cl | H | Cl | Im | 60-2 |
| 17 | 2,4-$(CH_3)_2$ | 4-Cl | H | Cl | Tr | 259 (Z) 3) |
| 18 | 2,4-$(CH_3)_2$ | 4-Cl | H | Cl | Im | 211 (Z) 3) |
| 19 | H | 3-Cl | H | Cl | Tr | Oel |
| 20 | H | 4-$OCH_3$ | H | Cl | Tr | Oel |
| 21 | 2,4-$(CH_3)_2$ | 3,4$(OCH_3)_2$ | H | Cl | Tr | Oel |
| 22 | H | 2,4-$Cl_2$ | H | Cl | Tr | 85-8 |
| 23 | H | 2,4-$Cl_2$ | H | Cl | Im | Oel |
| 24 | H | 3,4-$Cl_2$ | H | Cl | Tr | 221-3/0,05 |
| 25 | H | 3,4-$Cl_2$ | H | Cl | Im | 232/0,04 |
| 26 | 4-$CH_3$ | 3,4-$Cl_2$ | H | Cl | Tr | 272 (Z) 3) |
| 27 | 4-$CH_3$ | 3,4-$Cl_2$ | H | Cl | Im | 258 (Z) 3) |
| 28 | H | H | $CH_3$ | Cl | Tr | Oel |
| 29 | 4-$CH_3$ | 4-Cl | H | Cl | Pyr | 200-8/0,015 |
| 30 | H | 2-Br,4-Cl | H | Cl | Tr | Oel |
| 31 | H | 2-Br,4-Cl | H | Cl | Tr | 79 (Z) 4) |
| 32 | H | 2-F | H | Cl | Tr | Oel |

1) Im = Imidazol; Tr = Triazol; Pyr = Pyrazol 2) als Nitrat

3) als Salz mit 1/2 1,5-Naphthalindisulfonsäure 4) als Komplex mit 1/2 $CuCl_2$

...

0090269

B. Biologische Beispiele

Beispiel I

Weizenpflanzen werden im 3-Blattstadium mit Konidien des Weizenmehltaus (Erysiphe graminis) stark inokuliert und in einem Gewächshaus bei 20 °C und einer relativen Luftfeuchte von 90 bis 95 % aufgestellt. 3 Tage nach Inokulation werden die Pflanzen mit den zu prüfenden Verbindungen in den Wirkstoffkonzentrationen von 500, 250, 125, 60, 30 und 15 mg/Liter Spritzbrühe tropfnaß gespritzt. Nach einer Inkubationszeit von 10 Tagen untersucht man die Pflanzen auf Befall mit Weizenmehltau.

Der Befallsgrad wird ausgedrückt in % befallener Blattfläche, bezogen auf unbehandelte, infizierte Kontrollpflanzen (= 100 % Befall). Die Ergebnisse zeigt die nachstehende Tabelle I.

Tabelle I

| Verbindung gemäß Beispiel Nr. | mit Weizenmehltau befallene Blattfläche in % bei ... mg Wirkstoff/Liter Spritzbrühe | | | | | |
|---|---|---|---|---|---|---|
| | 500 | 250 | 125 | 60 | 30 | 15 |
| 1 | 0 | 0 | 0 | 0 | 0 | 0 |
| 5 | 0 | 0 | 0 | 0 | 0 | 0-3 |
| 7 | 0 | 0 | 0 | 0 | 0 | 0-3 |
| 8 | 0 | 0 | 0 | 0 | 0 | 0 |
| 22 | 0 | 0 | 0 | 0 | 0 | 0 |
| 10 | 0 | 0 | 0 | 0 | 0 | 0 |
| 11 | 0 | 0 | 0 | 0 | 0 | 0 |
| 12 | 0 | 0 | 0 | 0 | 0 | 0 |
| 2 | 0 | 0 | 0 | 0 | 0 | 0-3 |
| unbehandelte, infizierte Pflanzen | 100 | | | | | |

...

0090269

Beispiel II

Gerstenpflanzen werden im 3-Blattstadium mit Konidien des Gerstenmehltaus (Erysiphe graminis sp. hordei) stark inokuliert und in einem Gewächshaus bei 20 °C und einer relativen Luftfeuchte von 90 bis 95 % aufgestellt. 3 Tage nach Inokulation werden die Pflanzen mit den zu prüfenden Verbindungen in den Wirkstoffkonzentrationen von 500, 250, 125, 60 und 30 mg/Liter Spritzbrühe tropfnaß gespritzt. Nach einer Inkubationszeit von 10 Tagen wird auf Befall mit Gerstenmehltau untersucht. Der Befallsgrad wird ausgedrückt in % befallener Blattfläche, bezogen auf unbehandelte, infizierte Kontrollpflanzen (= 100 % Befall). Die Ergebnisse zeigt Tabelle II.

Tabelle II

| Verbindung gemäß Beispiel Nr. | mit Gerstenmehltau befallene Blattfläche in % bei ... mg Wirkstoff/Liter Spritzbrühe | | | | |
|---|---|---|---|---|---|
| | 500 | 250 | 125 | 60 | 30 |
| 1 | 0 | 0 | 0 | 0 | 0 |
| 5 | 0 | 0 | 0 | 0 | 0 |
| 7 | 0 | 0 | 0 | 0 | 0 |
| 8 | 0 | 0 | 0 | 0 | 0 |
| 22 | 0 | 0 | 0 | 0 | 0 |
| 10 | 0 | 0 | 0 | 0 | 0 |
| 11 | 0 | 0 | 0 | 0 | 0 |
| 12 | 0 | 0 | 0 | 0 | 0 |
| 2 | 0 | 0 | 0 | 0 | 0 |
| unbehandelte, infizierte Pflanzen | 100 | | | | |

...

Beispiel III

Gurkenpflanzen (Sorte Delikateß) werden im 2-Blattstadium mit einer Konidiensuspension von Gurkenmehltau (Erysiphe cichoracearum) stark inokuliert. Nach einer Antrocknungszeit der Sporensuspension von 30 Minuten werden die Pflanzen in einem Gewächshaus bei 22 °C und 90 % relativer Luftfeuchte aufgestellt. 3 Tage nach Infektion wird mit den zu prüfenden Verbindungen in den in Tabelle III angegebenen Wirkstoffkonzentrationen tropfnaß gespritzt. Nach 10 Tagen erfolgt die Bonitur. Der Befallsgrad wird ausgedrückt in % befallener Blattfläche, bezogen auf unbehandelte, infizierte Kontrollpflanzen (= 100 % Befall).
Tabelle III zeigt die Ergebnisse.

Tabelle III

| Verbindung gemäß Beispiel Nr. | mit Gurkenmehltau befallene Blattfläche in % bei ... mg Wirkstoff/Liter Spritzbrühe | | | | |
|---|---|---|---|---|---|
| | 500 | 250 | 125 | 60 | 30 |
| 1 | 0 | 0 | 0 | 0 | 0 |
| 5 | 0 | 0 | 0 | 0 | 0-3 |
| 7 | 0 | 0 | 0 | 0 | 0-3 |
| 8 | 0 | 0 | 0 | 0 | 0-3 |
| 22 | 0 | 0 | 0 | 0 | 0 |
| 10 | 0 | 0 | 0 | 0 | 0 |
| 11 | 0 | 0 | 0 | 0 | 0 |
| 12 | 0 | 0 | 0 | 0 | 0 |
| 2 | 0 | 0 | 0 | 0 | 0 |
| unbehandelte, infizierte Pflanzen | 100 | | | | |

...

Beispiel IV

Apfelunterlagen der Sorte EM IX werden im 4-Blattstadium mit einer Konidiensuspension von Apfelmehltau (Podosphaera leucotricha) stark infiziert. Anschließend kommen die Pflanzen für 16 Stunden in eine Klimakammer mit 20 °C und einer relativen Luftfeuchte von ca. 100 %. Danach werden sie in einem Gewächshaus bei 22 °C und einer relativen Luftfeuchte von 85 % aufgestellt. 3 Tage nach Infektion werden die Pflanzen mit den zu prüfenden Verbindungen in den in Tabelle IV genannten Wirkstoffkonzentrationen tropfnaß gespritzt. Nach 2 bis 3 Wochen wird der Mehltaubefall bonitiert und der Befallsgrad ausgedrückt in % befallener Blattfläche, bezogen auf unbehandelte, infizierte Kontrollpflanzen (= 100 % Befall). Die Ergebnisse sind in Tabelle IV zusammengefaßt.

Tabelle IV

| Verbindung gemäß Beispiel Nr. | mit Apfelmehltau befallene Blattfläche in % bei ... mg Wirkstoff/Liter Spritzbrühe | | | | |
|---|---|---|---|---|---|
| | 500 | 250 | 125 | 60 | 30 |
| 7 | 0 | 0 | 0 | 0 | 0 |
| 22 | 0 | 0 | 0 | 0 | 0 |
| 10 | 0 | 0 | 0 | 0 | 0 |
| 12 | 0 | 0 | 0 | 0 | 0 |
| unbehandelte, infizierte Pflanzen | 100 | | | | |

...

Beispiel V

Apfelunterlagen (EM IX) spritzt man im 4-Blattstadium mit den zu prüfenden Verbindungen in den Anwendungskonzentrationen von 500, 250, 125 und 60 mg Wirkstoff/Liter Spritzbrühe tropfnaß. Nach dem Antrocknen des Wirkstoffbelages wurden die Pflanzen sodann mit Konidien des Apfelschorfs (Venturia inaequalis) stark infiziert und tropfnaß in eine Klimakammer gestellt, deren Temperatur 22 °C und deren relative Luftfeuchtigkeit 100 % betrug. Nach einer Infektionszeit von 48 Stunden kamen die Pflanzen in ein Gewächshaus mit 18 °C und einer relativen Luftfeuchte von 95 bis 100 %. Nach einer Inkubationszeit von 14 Tagen wurden die Pflanzen auf Befall mit Apfelschorf (Venturia inaequalis) untersucht. Die Beurteilung des Befalls erfolgte wie üblich nach Augenschein. Der Befallsgrad wurde in % befallener Blattfläche, bezogen auf unbehandelte, infizierte Pflanzen, ausgedrückt und ist in Tabelle V wiedergegeben.

Tabelle V

| Verbindung gemäß Beispiel Nr. | % Schorfbefall bei ... mg Wirkstoff/Liter Spritzbrühe | | | |
|---|---|---|---|---|
| | 500 | 250 | 125 | 60 |
| 1 | 0 | 0 | 0 | 0 |
| 22 | 0 | 0 | 0 | 0 |
| 10 | 0 | 0 | 0 | 0 |
| 11 | 0 | 0 | 0 | 0 |
| 12 | 0 | 0 | 0 | 0 |
| unbehandelte, infizierte Pflanzen | 100 | | | |

...

Beispiel VI

Zuckerrübenpflanzen wurden im 6-Blattstadium mit den zu prüfenden Verbindungen in den Aufwandmengen von 500, 250, 125 und 60 mg/l Spritzbrühe behandelt.

Nach Antrocknen des Wirkstoffbelages wurden die Pflanzen mit Konidien des Erregers der Blattfleckenkrankheit der Rübe (Cercospora beticola) stark inokuliert und tropfnaß in eine Klimakammer mit ca. 100 % relativer Luftfeuchte und 25 °C gestellt. 14 Tage später wurden sie auf Befall mit der Blattfleckenkrankheit untersucht. Der Befallsgrad wurde in % befallener Blattfläche, bezogen auf unbehandelte, infizierte Kontrollpflanzen (= 100 %), ausgedrückt. Die Ergebnisse zeigt Tabelle VI.

Tabelle VI

| Verbindung gemäß Beispiel Nr. | mit Cercospora beticola befallene Blattfläche in % bei ...mg Wirkstoff/Liter Spritzbrühe | | | |
|---|---|---|---|---|
| | 500 | 250 | 125 | 60 |
| 1 | 0 | 0 | 0 | 0 |
| 22 | 0 | 0 | 0 | 0 |
| 10 | 0 | 0 | 0 | 0 |
| 12 | 0 | 0 | 0 | 0-3 |
| unbehandelte, infizierte Pflanzen | 100 | | | |

...

Beispiel VII

Weizenpflanzen wurden mit den zu prüfenden Verbindungen in den in Tabelle VII genannten Anwendungskonzentrationen behandelt. Nach dem Antrocknen des Wirkstoffbelages wurden die Pflanzen mit Sporen des Weizenbraunrostes (Puccinia triticina) inokuliert und tropfnaß in eine Klimakammer bei 20 °C und 100 % relativer Luftfeuchte gestellt. 24 Stunden später kamen die Pflanzen in ein Gewächshaus zurück und wurden hier 14 Tage nach Inokulation auf Befall mit Weizenbraunrost untersucht. Der Befallsgrad wurde ausgedrückt in % befallener Blattfläche, bezogen auf unbehandelte, infizierte Kontrollpflanzen (= 100 % Befall). Tabelle VII zeigt die gute Wirkung der untersuchten Verbindungen.

Tabelle VII

| Verbindung gemäß Beispiel Nr. | % mit Braunrost befallene Blattfläche bei ... mg Wirkstoff/Liter Spritzbrühe | | | |
|---|---|---|---|---|
| | 500 | 250 | 125 | 60 |
| 6 | 0 | 0 | 0 | 0 |
| 22 | 0 | 0 | 0 | 0 |
| 10 | 0 | 0 | 0 | 0 |
| unbehandelte, infizierte Pflanzen | 100 | | | |

...

0090269



Patentansprüche

1. 3-Azolyl-1,2-diaryl-1-halogen-1-propene der Formel (I),

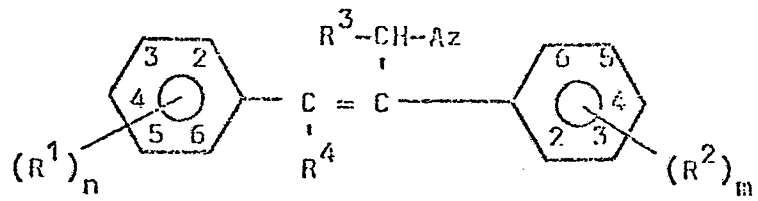

(I),

in welcher

$R^1$ und $R^2$ unabhängig voneinander für Wasserstoff, Halogen, $-CF_3$, $-OCF_2CF_2H$, $C_1$- bis $C_8$-Alkyl, $C_5$- oder $C_6$-Cycloalkyl, $C_1$- bis $C_6$-Alkoxi, $C_2$- bis $C_6$-Alkenoxi, oder auch für Phenoxi oder Phenyl und die Halogensubstitutionsprodukte der beiden letztgenannten Reste stehen oder Reste der Formeln $-CH_2CH_2CH_2-$, $-CH_2CH_2CH_2CH_2-$ oder $-CH=CH-CH=CH-$, die die C-Atome der Ringpositionen 2 und 3 oder 3 und 4 miteinander verbinden, bedeuten,

$R^3$ Wasserstoff oder ein $C_1$- bis $C_5$-Alkylrest ist,

$R^4$ für Chlor oder Brom steht,

n und m 1, 2 oder 3 sind, wobei bei Mehrfachsubstitution auch unterschiedliche Substituenten in Frage kommen, und

Az die Bedeutung von 1,2,4-Triazol-1-yl oder von Imidazol-1-yl oder von Pyrazol-1-yl hat,

sowie deren Salze, Komplexsalze und Quaternisierungsprodukte.

BAD ORIGINAL

2. Verfahren zur Herstellung der in Anspruch 1 definierten Verbindungen der Formel (I), dadurch gekennzeichnet, daß man 1 Mol eines 1,2-Diaryl-1,3-dihalogen-1-propens der allgemeinen Formel (II),

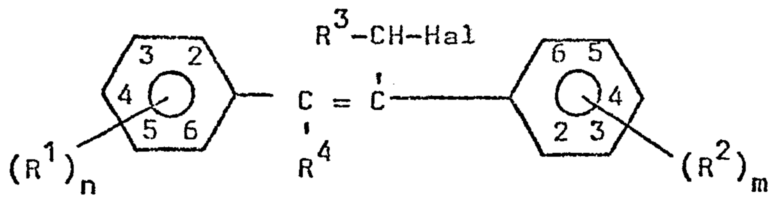

(II),

in der $R^1$, $R^2$, $R^3$ und $R^4$ die in Anspruch 1 angegebenen Bedeutungen haben und Hal für Chlor oder Brom steht mit der 1,0- bis 1,2fach molaren Menge 1,2,4-Triazol, Imidazol oder Pyrazol in Gegenwart eines Lösungsmittels und in Anwesenheit von mindestens 1 Mol einer Base bei Temperaturen zwischen 0 und 120 °C umsetzt.

3. Biozide Zubereitungen, in denen mindestens eine Verbindung nach Anspruch 1 enthalten ist.

4. Verwendung der Verbindungen nach Anspruch 1 als Fungizide und Bakterizide.

5. Verfahren zur Bekämpfung von schädlichen Pilzen und Bakterien im Obst-, Gemüse-, Getreide- und Zierpflanzenanbau, im Rahmen der Holzkonservierung, sowie in Anstrichfarben, Kühlschmiermitteln, Bohr- und Schneidölen und anderen technischen Präparaten, dadurch gekennzeichnet, daß hierfür eine Zubereitung eingesetzt wird, die mindestens eine der vom Anspruch 1 umfaßten Verbindungen enthält.

0090269

HOE 82/F 800

Patentansprüche für Österreich

1. Verfahren zur Herstellung von 3-Azolyl-1,2-diaryl-1-halogen-1-propenen der Formel (I)

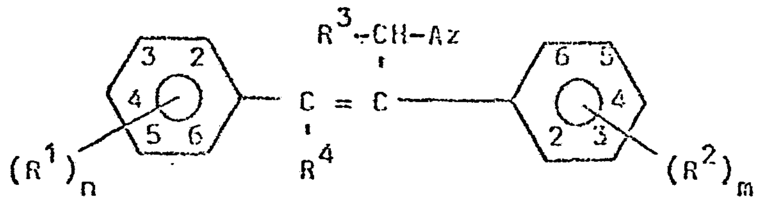

(I),

in welcher

$R^1$ und $R^2$ unabhängig voneinander für Wasserstoff, Halogen, $-CF_3$, $-OCF_2CF_2H$, $C_1$- bis $C_8$-Alkyl, $C_5$- oder $C_6$-Cycloalkyl, $C_1$- bis $C_6$-Alkoxi, $C_2$- bis $C_6$-Alkenoxi, oder auch für Phenoxi oder Phenyl und die Halogensubstitutionsprodukte der beiden letztgenannten Reste stehen oder Reste der Formeln $-CH_2CH_2CH_2-$, $-CH_2CH_2CH_2CH_2-$ oder $-CH=CH-CH=CH-$, die die C-Atome der Ringpositionen 2 und 3 oder 3 und 4 miteinander verbinden, bedeuten,

$R^3$ Wasserstoff oder ein $C_1$- bis $C_5$-Alkylrest ist,

$R^4$ für Chlor oder Brom steht,

n und m 1, 2 oder 3 sind, wobei bei Mehrfachsubstitution auch unterschiedliche Substituenten in Frage kommen, und

Az die Bedeutung von 1,2,4-Triazol-1-yl oder von Imidazol-1-yl oder von Pyrazol-1-yl hat,

sowie deren Salze, Komplexsalze und Quaternisierungsprodukte,

dadurch gekennzeichnet, daß man 1 Mol eines 1,2-Diaryl-1,3-dihalogen-1-propens der allgemeinen Formel (II),

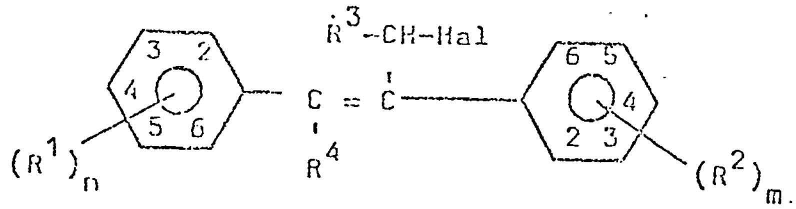

(II),

in der $R^1$, $R^2$, $R^{3'}$ und $R^4$ die oben angegebenen Bedeutungen haben und Hal für Chlor oder Brom steht mit der 1,0- bis 1,2fach molaren Menge 1,2,4-Triazol, Imidazol oder Pyrazol in Gegenwart eines Lösungsmittels und in Anwesenheit von mindestens 1 Mol einer Base bei Temperaturen zwischen 0 und 120 °C umsetzt, worauf man - sofern gewünscht - die erhaltenen, basisch reagierenden Verbindungen in an sich bekannter Weise in Salze, Komplexsalze oder Quaternisierungsprodukte überführt.

2. Biozide Zubereitungen, in denen mindestens eine der nach Anspruch 1 erhaltenen Verbindungen enthalten ist.

3. Verfahren zu Bekämpfung von schädlichen Pilzen und Bakterien im Obst-, Gemüse-, Getreide- und Zierpflanzenanbau, im Rahmen der Holzkonservierung, sowie in Anstrichfarben, Kühlschmiermitteln, Bohr- und Schneidölen und anderen technischen Präparaten, dadurch gekennzeichnet, daß hierfür eine Zubereitung eingesetzt wird, die mindestens eine der vom Anspruch 1 umfaßten Verbindungen enthält.

BAD ORIGINAL

Europäisches Patentamt

**EUROPÄISCHER RECHERCHENBERICHT**

0090269 Nummer der Anmeldung

EP 83 10 2618

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | DE-A-2 652 313 (BASF) * Ansprüche * ----- | 1-5 | C 07 D 249/08<br>C 07 D 233/56<br>C 07 D 231/12<br>A 01 N 43/50<br>A 01 N 43/56<br>A 01 N 43/64 |
| | | | RECHERCHIERTE SACHGEBIETE (Int. Cl. 3) |
| | | | C 07 D 233/00<br>C 07 D 249/00<br>C 07 D 231/00 |

Der vorliegende Recherchenbericht wurde für alle Patentansprüche erstellt.

| Recherchenort | Abschlußdatum der Recherche | Prüfer |
|---|---|---|
| DEN HAAG | 07-07-1983 | CREMERS K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03 82